# EUROPEAN PATENT SPECIFICATION

(11) **EP 3 643 294 B1**
(45) Date of publication and mention of the grant of the patent: **29.11.2023**
(21) Application number: 17914773.1
(22) Date of filing: 19.06.2017
(51) Int. Cl.: A61K 8/60, A61K 8/02, A61K 8/73, A61K 8/86, A61Q 1/14, A61Q 5/02, A61Q 5/12, A61Q 17/04, A61Q 19/00, A61Q 19/10

(54) **TABLET-TYPE FREEZE-DRIED COSMETIC**
TABLETTENFÖRMIGES GEFRIERTROCKNTES KOSMETIKUM
COSMÉTIQUE LYOPHILISÉ DE TYPE COMPRIMÉ

(43) Date of publication of application: 29.04.2020
(73) Proprietor: Shiseido Company, Ltd., Tokyo 104-0061 (JP)
(72) Inventor: KIKUTA, Masayuki, Yokohama-shi Kanagawa 224-8558 (JP)
(74) Representative: Ter Meer Steinmeister & Partner
(86) International application number: PCT/JP2017/022595
(87) International publication number: WO 2018/235147

(56) References cited:
- EP-A2- 0 412 449
- JP-A- H0 368 507
- JP-A- H08 507 064
- JP-A- S61 180 800
- JP-A- 2006 509 525
- JP-A- 2015 224 219
- JP-A- 2017 048 303
- JP-A- 2017 110 001
- DATABASE GNPD [Online] MINTEL; 14 May 2015 (2015-05-14), anonymous: "Human Essence", XP055781506, Database accession no. 3185575
- DATABASE GNPD [Online] MINTEL; 28 June 2016 (2016-06-28), anonymous: "Intense Repair Freeze-Dried Flocculant Treatment", XP055781509, Database accession no. 4098653
- DATABASE GNPD [Online] MINTEL; 29 December 2016 (2016-12-29), anonymous: "Lamellar Essence C", XP055781512, Database accession no. 4510523
- DATABASE WPI Week 201745 Thomson Scientific, London, GB; AN 2017-370600 XP002802252, & CN 106 726 703 A (XU H) 31 May 2017 (2017-05-31)
- DATABASE WPI Week 201472 Thomson Scientific, London, GB; AN 2014-T02086 XP002802253, & CN 103 961 283 A (GUANGZHOU SHUTAI BIOLOGICAL TECHNOLOGY C) 6 August 2014 (2014-08-06)

## Description

### FIELD OF THE INVENTION

The present invention relates to a tablet-type freeze-dried cosmetic, and particularly to improvement in easiness of handling of the cosmetic as a tablet.

### BACKGROUND OF THE INVENTION

Highly functional skin care cosmetics blended with active components are usually provided in forms of cosmetic lotions or beauty serums, and their effect is exhibited by using them daily and customary.

On the other hand, products with less burden of portability are demanded in recent years since opportunities for performing skin care at travel destinations, business trip destinations, or sports gyms are increasing. Furthermore, senses of beauty are increasing in recent years, and thus high-quality products that can achieve satisfaction equal to or higher than daily care are demanded for skin care cosmetics to be used away from home.

Furthermore, there is a high demand for products that can be used easily and customized not only in portable cosmetics as described above, but also in skin care cosmetics to be used daily or in accordance with the condition of skin.

As one means to meet such demand, liquid compositions such as cosmetic lotions may be formulated as dried preparations to be used by redissolving in water upon use.

As a technique related to formulation of dried preparations of liquid compositions, Patent Literature 1 discloses that L-ascorbic acid 2-phosphate that is unstable in the coexistence with water is blended with a sugar alcohol, a low hygroscopic oligosaccharide, and a water-soluble polymer to give an aqueous solution, and the aqueous solution is freeze-dried so that it can be stably maintained until use, for example.

Furthermore, Patent Literature 2 discloses that a freeze-dried cosmetic obtained by freeze-drying an aqueous solution blended with sodium hyaluronate, collagen, and an ascorbic acid derivative does not contract or break by moisture absorption or change in quality by bacteria or oxidation after long-time storage, and that it dissolves extremely quickly when redissolved in water or cosmetic lotions upon use.

Furthermore, Patent Literature 3 discloses that a hyaluronic acid solid composition that is stable at room temperature is obtained by freeze-drying an aqueous solution comprising sodium hyaluronate and saccharides.

Furthermore, Patent Literature 4 discloses that a powder or a granular cosmetic that uses freeze-dried products of an aqueous solution comprising hyaluronic acid or sodium hyaluronate and L-ascorbyl magnesium phosphate improves solubility of both components with water.

### CITATION LIST

### PATENT LITERATURES

PATENT LITERATURE 1: Japanese Unexamined Patent Publication No. 2004-149468 A
PATENT LITERATURE 2: Japanese Unexamined Patent Publication No. 2006-182750 A
PATENT LITERATURE 3: Japanese Unexamined Patent Publication No. 2003-95955 A
PATENT LITERATURE 4: Japanese Unexamined Patent Publication No. H2-215707 A

### DISCLOSURE OF THE INVENTION

### PROBLEM TO BE SOLVED BY THE INVENTION

The freeze-dried compositions taught in Patent Literatures 1 to 4, however, were not satisfactory in terms of releasability from containers and resolubility as freeze-dried products formed in tablet shapes and feeling in use of the redissolved solution. Therefore, it was difficult to produce the compositions as tablet-type cosmetics that are highly portable and easy to use. In particular, a technique related to tablets having low hygroscopicity during storage and solubility such that the tablet dissolves (in water) instantaneously when water is added (poured on the tablet) is not known yet.

The present invention has been made in view of the above-mentioned circumstances, and an object thereof is to provide a tablet-type freeze-dried cosmetic that is easy to handle.

### MEANS TO SOLVE THE PROBLEM

The present inventors have diligently investigated on the above-mentioned problem, and as a result, they have found that a composition that comprises a specific molecular amount of polyethylene glycol and trehalose and is formed in a tablet form by freeze-drying so that it becomes a prescribed bulk specific gravity dissolves instantaneously by addition of water although it is low in hygroscopicity, has high-releasablity, and is easy to handle, and completed the present invention.

That is, a freeze-dried tablet cosmetic according to the present invention comprises:
(A) 30 to 91% by mass of trehalose;
(B) 4.0 to 16% by mass of polyethylene glycol having a molecular weight of 11000 to 25000;
(C) 0.01 to 2% by mass of hyaluronic acid; and
(D) 0.01 to 1.0% by mass of a thickener selected from a group consisting of xanthan gum, tamarind gum, sodium alginate, gellan gum, agar, polyvinyl alcohol, carboxyvinyl polymer, and quince seed,
wherein a bulk specific gravity is 0.1 to 0.5 g/ml.

In the cosmetic, it is preferable that the (B) comprises polyethylene glycol having a number average molecular weight of 15000 to 20000.

The cosmetic comprises: (C) hyaluronic acid, and (D) a thickener.

In the cosmetic, the hyaluronic acid (C) is blended at 0.01 to 2% by mass.

In the cosmetic, the thickener (D) is blended at 0.01 to 1.0% by mass.

In the cosmetic, the thickener (D) is selected from xanthan gum, tamarind gum, sodium alginate, gellan gum, agar, polyvinyl alcohol, carboxyvinyl polymer, and quince seed.

### EFFECT OF THE INVENTION

According to the present invention, a tablet-type freeze-dried cosmetic that is applicable to skin and can dissolve instantaneously when a small amount of water is added can be obtained by blending trehalose and a prescribed number average molecular weight of polyethylene glycol. Furthermore, the tablet-type cosmetic is extremely low in hygroscopicity and enables to provide cosmetics having portability and easiness.

Furthermore, a tablet-type freeze-dried cosmetic excellent in usability can be obtained when hyaluronic acid and a thickener are blended.

### BEST MODE FOR CARRYING OUT THE INVENTION

The present invention will be described in detail hereinbelow.

A tablet-type freeze-dried cosmetic according to the present invention comprises trehalose and a specific number average molecular weight of polyethylene glycol and is basically produced by volatilizing a medium from a uniform solution/dispersion of the components. First of all, the constituents of the present invention will be described.

### <Diluents>

Components blended to the present invention as diluents are: (A) trehalose, and (B) polyethylene glycol.

### (A) Trehalose

Trehalose (A) used in the present invention is a disaccharide of which two D-glucoses are bonded. There are three kinds of bonding forms such as: α,α-; α,β-; and β,β-. The general bonding form is α,α- which is recognized to exist naturally.

Trehalose is one kind of saccharides and has a high-water-retention capacity. Therefore, it is excellent in stability, resolubility and releasability as a freeze-dried cosmetic compared to saccharides such as erithritol, xylitol, sorbitol and the like.

The blending amount of trehalose is 30 to 91% by mass in the cosmetic. Furthermore, when it is 65 to 91% by mass, it is more preferable since it is excellent in high-temperature stability. When it is less than 30% by mass, it is not preferable since high-temperature stability and solubility to water upon redissolving may not be sufficient. Furthermore, when it exceeds 91% by mass, high-temperature stability may be excellent, but it is not preferable since it may hardly freeze and bumping may occur, or in terms of feeling in use.

Examples of commercially available products of trehalose include, but not limited to, Trehalose (for cosmetics) (available from HAYASHIBARA CO., LTD).

Saccharides other than trehalose may be added within a range of not adversely affecting the present invention.

Examples of other saccharides include, but not limited to, maltose, maltotetraose, and maltosyltrehalose.

### (B) Polyethylene glycol

Examples of the polyethylene glycol (B) used in the present invention include those that have a number average molecular weight of 11000 to 20000 in terms of shaping property of the tablet. It is more preferable when it is 15000 to 20000 in terms of formability and releasability.

When the number average molecular weight is less than 11000, it is not preferable since releasability may be deteriorated. Furthermore, when the number average molecular weight is greater than 20000, it is not preferable since resolubility may be poor and feeling in use may be inferior.

The blending amount of the polyethylene glycol (B) is 4 to 16% by mass, and more preferably 12 to 16% by mass in the cosmetic. When the blending amount exceeds 16% by mass, releasability and feeling in use of the tablet may deteriorate.

Furthermore, the polyethylene glycol that is synthesized in publicly known synthesizing method, or commercially available products may be used.

Examples of commercially available products of the polyethylene glycol include, but not limited to, PEG-20000 (available from Sanyo Chemical Industries, Ltd.).

### (C) Hyaluronic acid

The Hyaluronic acid (C) used in the present invention is a linear polymer of which N-acetyl-D-glucosamine residue and D-glucuronic acid residue are bonded alternately, and those that are processed in form of powder can be used.

The hyaluronic acid can be obtained by isolating and extracting from cockscombs or other animal tissue, or by a fermentation method that uses microorganisms such as Streptococcus, for example. Furthermore, hyaluronic acid metal salts such as hyaluronic acid sodium salt and hyaluronic acid potassium salt or powders of hyaluronic acid derivatives obtained by etherification, esterification, amidation, acetylation, acetalization, or ketalization of hydroxyl or carboxyl groups of hyaluronic acid may be used as a hyaluronic acid derivative in the present invention.

Furthermore, commercially available products can be used as hyaluronic acid. Examples thereof include, but not limited to, Hyaluronsan HA-LQ (available from Kewpie Fine Chemicals Corporation) and Hyaluronic Acid FCH (available from Kikkoman Biochemifa Company).

Furthermore, the molecular amount of the hyaluronic acid (C) is not limited in particular, but the molecular amount of 100,000 or more is preferable, and approximately 500,000 to 3,000,000 is more preferable. When hyaluronic acid having a low molecular weight of less than 100,000 is used, shaping property and feeling in use after redissolution of the tablet may not be sufficient. Furthermore, when the molecular weight is greater than 3,000,000, it is not preferable since solubility and feeling in use after redissolution may not be sufficient.

In particular, among the abovementioned diluents, the hyaluronic acid (C) shows extremely high viscosity in form of a low concentrated aqueous solution. Therefore, the blending amount the hyaluronic acid is highly related to properties of the freeze-dried tablet and the redissolved solution thereof.

The blending amount of the hyaluronic acid (C) is 0.01 to 2% by mass, and more preferably 0.05 to 0.2% by mass in the cosmetic. When the blending amount exceeds 2% by mass, it is not preferable since solubility and releasability of the tablet deteriorate.

### (D) Thickener

The thickener (D) used in the present invention is used to enhance formability and releasability of the tablet cosmetic.

Water-soluble polymers such as xanthan gum, tamarind gum, sodium alginate, gellan gum, agar, polyvinyl alcohol, carboxyvinyl polymer, and quince seed are used as the thickener (D). Among the above, xanthan gum, tamarind gum, and quince seed are used preferably since they are less sticky or tight when the tablet cosmetic is used and dried.

The blending amount of the thickener (D) is 0.01 to 1.0% by mass, and more preferably 0.05 to 0.1% by mass in the cosmetic. When the blending amount exceeds 1.0% by mass, releasability and feeling in use of the tablet may deteriorate. When the blending amount is less than 0.01% by mass, formability and releasability may deteriorate.

Examples of commercially available products of such thickeners include, but not limited to, xanthan gum (KELTROL^{®}, available from CP Kelco), tamarind gum (GLYLOID^{®} 6C, available from Dainippon Pharmaceutical Co., Ltd.), sodium alginate (Duck Algin NSPH, available from Kikkoman Biochemifa Company), gellan gum (KELCOGEL^{®}, available from DSP GOKYO FOOD & CHEMICAL Co., Ltd.), and agar (Ina Agar CS-110, available from Ina Food Industry Co., Ltd.).

In addition to the above-mentioned components, the tablet-type freeze-dried cosmetic may comprise other components that can be generally blended to cosmetics and quasi drugs within a range of not adversely affecting the effect of the present invention.

Use of aqueous components is particularly preferable as other components upon production of the present invention, oily components can be blended by solubilizing with surfactants.

Examples of aqueous components include, but not limited to, polyhydric alcohols, water-soluble polymers other than the component (D), chelating agents, pH adjusters, and preservatives.

Examples of polyhydric alcohols include, but not limited to, ethylene glycol, propylene glycol, 1, 3-butylene glycol, glycerin, polyethylene glycol having molecular weights other than the above, and polyglycerin.

Examples of water-soluble polymers include, but not limited to: hydrophilic synthetic polymers such as polyacrylic acid, polyethylene glycol, polyacrylamide, polyalkylacrylamide/polyacrylamide copolymer, carboxymethyl cellulose, cationized cellulose, pluronic, macrogol, povidone, polyvinyl methacrylate, and polyethyleneimine; hydrophilic natural polymers such as succinoglycan, guar gum, locust bean gum, curdlan, alginic acid, carrageenan, mannan, pectin, gum arabic, karaya gum, casein, α-cyclodextrin, dextrin, dextran, gelatin, collagen, pectin, starch, chitin and derivatives thereof, chitosan and derivatives thereof, elastin, heparin, heparan sulfate, methylcellulose, hydroxyethyl cellulose, hydroxypropyl cellulose, and hydroxypropyl methylcellulose; and hydrophilic clay minerals such as laponite, bentonite, and smectite.

Examples of chelating agents include, but not limited to: 1-hydroxyethane-1,1-diphosphonic acid; 1- hydroxyethane-1,1-diphosphonic acid tetrasodium salt; disodium edetate; trisodium edetate; tetrasodium edetate; sodium citrate; sodium polyphosphate; sodium metaphosphate; gluconic acid; phosphoric acid; citric acid; ascorbic acid; succinic acid; edetic acid; and HEDTA-3Na.

Examples of pH adjusters include, but not limited to, buffers such as lactic acid-sodium lactate, citric acid-sodium citrate, and succinic acid-sodium succinate.

Examples of preservatives include, but not limited to, p-hydroxybenzoate ester, and phenoxyethanol.

Examples of oily components include, but not limited to, perfumes, and oily agents (e.g. vitamin A).

Examples of other components that can be blended include, but not limited to, active components such as: moisturizers (for example: PEG/PPG dimethyl ether such as 2-methacryroyloxyethylphosphorylcholine (MPC) copolymer and PEG/PPG-14/7 dimethyl ether, water-soluble collagen, hydrogen-peroxide-treated yeast hydrolysate, sodium dl-pyrrolidonecarboxylate, L-hydroxyproline, calcium chloride, and magnesium chloride); various extracts (for example: rose, phellodendron bark, coptis japonica, lithospermum root, peony, Swertia japonica, birch, sage, loquat, carrot, aloe, mallow, iris, grape, coix seed, sponge gourd, lily, saffron, Cnidium officinale, ginger root, St. John's wort, Ononis, garlic, red pepper, chinpi, Angelica acutiloba and seaweed); and other water-soluble agents (for example, tranexamic acid). Desired effects can be provided to the cosmetic of the present invention by blending these components.

The thickener and trehalose that are the essential components can also provide moisturizing effect to the present invention.

The tablet-type freeze-dried cosmetic according to the present invention can be obtained as a dried product that remains after dissolving or dispersing the above-mentioned blending components to a volatile liquid medium such as water or lower alcohol to prepare a before-drying solution, freezing and then decompressing the solution to sublimate.

Upon production of the tablet-type freeze-dried cosmetic according to the present invention, the diluent component dissolved to a medium such as water forms a three-dimensional network structure in the medium. When only the medium is to be removed from such state, the network structure may shrink, deform or collapse due to drying, and thus it is usually difficult to maintain such structure. In the present invention, however, since trehalose that is blended together with the diluent supports the network structure instead of the medium after volatilization, it is considered that the structure formed by the diluent is mostly maintained.

Furthermore, the dried product that maintains the three-dimensional network structure formed by the diluent is in a porous state of which the medium part is removed from the structure. When water is added upon redissolution, water permeates from each pore to the inner side of the network structure, and dissolving of the entire tablet agent proceeds. Here, the higher the density of the constituents including the structure is, the more difficult for water to permeate inside: thus, the preparation becomes difficult to dissolve. On the other hand, when the density of the constituents is too low, the preparation itself becomes fragile and broken easily, and tends to absorb moisture during storage.

Consequently, in order to make the preparation to be extremely low in hygroscopicity during storage while it has solubility such that water permeates instantaneously to collapse the preparation, the bulk density of the constituents needs to be set in a constant range.

Consequently, the tablet-type cosmetic according to the present invention needs to comprise the above-mentioned constituents and the bulk specific gravity thereof needs to be 0.1 to 0.5 g/ml, and preferably 0.15 to 0.3 g/ml.

When the bulk specific gravity is less than 0.1 g/ml, hygroscopicity during storage tends to be higher. When it exceeds 0.5 g/ml, solubility to water tends to be lowered. On the other hand, when the bulk specific gravity is within the above-mentioned range, the number of pores of which the medium is removed becomes adequate, and the network structure of the thickener becomes fixed in a suitably widened state. Therefore, when it is redissolved by adding water, excellent solubility may be exhibited.

As a method to measure the bulk specific gravity, it is calculated by: (the weight of the freeze-dried tablet (g)) ÷ (the volume of the freeze-drying mold (ml)).

For example, the tablet-type cosmetic according to the present invention can be produced by: dissolving or dispersing the constituents to a volatile liquid medium such as water or lower alcohol to give a solution; filling a tablet-shaped mold with the solution; freeze-drying the same; and removing the tablet from the mold as necessary.

The present invention can be formed in any size and shape by setting the volume of the preparation and the amount of the constituents and the medium to fulfill the above-mentioned bulk specific gravity.

However, extremely thin or too complexed shapes are not preferable since it is problematic in formability.

Furthermore, the preparation of the present invention has low hygroscopicity and high releasability such that it can be easily released from the container. Therefore, it can be packed not only in forms of packing the tablet that is still filled in the container per each use (e.g. blister pack), but also in any form such as packing only the tablet that is taken out from the container (mold).

The tablet-type cosmetic according to the present invention can be used by adding a suitable amount of water to redissolve the preparation and applying the solution thereof to skin or the like. The addition amount of water may be adjusted to an extent that the preparation dissolves completely and a viscosity such that the solution can be applied easily. When it is too much, sufficient effect may not be achieved since the component concentration is too low. When it is too small, it may cause stickiness upon application. Therefore, although it is not limited in particular, it is preferable to adjust the amount of water to approximately 1 to 10 times the volume of the preparation.

The place to add water to the preparation may be on palms, on parts to be applied, or inside a suitable container. As a use method of the present invention, it is preferable to put the preparation onto the palm, add water to the preparation, and then apply the solution to parts to be applied by hand.

Cosmetic lotions may be used instead of water if the preparation can be redissolved.

The tablet-type cosmetic according to the present invention can be in product forms such as cosmetic lotions, serums, face masks, facial cleansers, facial cleansing foams, hand creams, shampoos, conditioners, hair treatments, sunscreens and the like depending on the components to be blended. In particular, use as cosmetic lotions or serums for moisturizing is preferable.

### EXAMPLES

The present invention will be described with reference to the following examples, but the present invention is not limited thereto. The blending amounts are expressed with "% by mass" with respect to the system in which the component is blended unless otherwise specified.

First of all, evaluation methods and criteria used in the following tests will be described.

### <Solubility>

The tablet-type freeze-dried cosmetic (approximately 100 mg) of each test example was placed on the palm, 500 µl of water was dripped thereto, and rubbed with fingers. The time required for the preparation to completely dissolve was evaluated based on the following criteria.
A: The preparation dissolved instantaneously and completely.
B: The preparation dissolved completely, but it took some time.
C: The preparation was slightly difficult to dissolve, and undissolved residues remained partially.
D: The preparation was difficult to dissolve, and undissolved residues remained.
E: The preparation did not dissolve.

### <Usability>

The tablet-type freeze-dried cosmetic (approximately 100 mg) of each test example was placed on the palm, and 500 µl of water was dripped thereto to dissolve the preparation. Feeling in use upon application to skin (moisture feeling, non-stickiness, smoothness of skin) was evaluated by 10 professional panelists based on the following criteria.
A: More than 6 panelists out of 10 panelists answered that feeling in use was good.
B: More than 3 and less than 6 panelists out of 10 panelists answered that feeling in use was good.
C: Less than 3 panelists out of 10 panelists answered that feeling in use was good.

### <Releasability>

Easiness in releasing when taking the dried product out from the container after the drying process in the production method of the tablet-type freeze-dried cosmetics of each test example was evaluated based on the following criteria.
A: The preparation was released from the container extremely easily.
B: The preparation was released from the container easily.
C: The preparation was slightly difficult to be released from the container.
D: The preparation was extremely difficult to be released from the container.

### <Formability>

Formability after the drying process in the production method of the tablet-type freeze-dried cosmetics of each test example was evaluated based on the following criteria.
A: The preparation was formed as the mold from the container.
B: The preparation was formed almost as the mold from the container.
C: The preparation was formed with small cracks from the container.
D: The preparation was not formed as the mold from the container: e.g. the preparation became smaller than the mold.
E: The preparation was formed with big cracks or was fragile and broken.

The test example compositions having the formulations described in the following tables were prepared in accordance with the following production method.

### <Production Method>

Each blending component was dissolved or dispersed to a volatile liquid medium such as water or lower alcohol to give a solution. The solution was poured into a mold, and after freezing, it was decompressed and sublimated to obtain the tablet-type freeze-dried cosmetic as the remaining dried product.

First of all, the present inventors investigated on formability of the tablet cosmetic with respect to saccharides used as the diluent.

The formulations of the test examples shown in the following Table 1 is as follows.

| | |
|---|---|
| Ion exchanged water | Balance |
| Saccharides | Blended at the amounts shown in Table 1 |
| Total | 100% by mass |

**[Table 1]**

| Blending amounts of each saccharide (%) | 1 | 2 | 5 | 10 | 20 |
|---|---|---|---|---|---|
| Trehalose | D | D | C | B | B |
| Maltitol | D | D | D | C | E |
| Sorbitol | E | E | E | E | E |
| Xylitol | E | E | E | E | E |

From the above, it was found that use of trehalose is superior in terms of formability as a basic skeleton of the diluent among the saccharides.

Therefore, the present inventors investigated on the blending amount of trehalose with respect to the solution before drying in the following. The blending amounts in Table 2 show the blending amounts of each component in the solution before drying, not in the freeze-dried cosmetic.

**[Table 2]**

| Test Example | 2-1 | 2-2 | 2-3 | 2-4 | 2-5 | 2-6 |
|---|---|---|---|---|---|---|
| Ion-exchanged water | 97 | 95 | 90 | 80 | 70 | 50 |
| Trehalose | 3 | 5 | 10 | 20 | 30 | 50 |
| Formability | D | C | B | B | E | E |
| Releasability | A | A | A | A | C | E |
| Resolubility | A | A | A | A | C | D |

As it can be seen from Test Examples 2-1 to 2-6, when the blending amount of trehalose with respect to the solution before drying exceeds 20% by mass, it is not preferable in terms of formability because bumping occurs. Furthermore, as for resolubility, when the blending amount of trehalose with respect to the solution before drying exceeds 20% by mass, it is not preferable because residues remain undissolved.

Accordingly, the present inventors investigated whether formability and releasability of the tablet-type cosmetic after freeze-drying can be improved by adding polyethylene glycol (B) to the solution before drying as the other component. The blending amounts in Table 3 show the blending amounts of each component in the solution before drying, not in the freeze-dried cosmetic.

**[Table 3]**

| Test Example | 2-3 | 2-4 | 3-1 | 3-2 |
|---|---|---|---|---|
| Ion-exchanged water | 90 | 80 | 88 | 78 |
| Trehalose (*1) | 10 | 20 | 10 | 20 |
| PEG-400 (Polyethylene glycol, number average molecular weight 20000) | - | - | 2 | 2 |
| Formability | B | B | A | A |
| Releasability | A | A | A | A |
| Resolubility | A | A | A | A |

### (*1) Trehalose; manufactured by HAYASHIBARA CO., LTD.

As it can be seen from Test Examples 3-1 to 3-2, it was found that when the polyethylene glycol (B) was added to the solution before drying, it becomes a diluent excellent in formability and releasability while maintaining resolubility.

The present inventors further investigated on usability as a tablet cosmetic.

**[Table 4]**

| Test Example | 4-1 (comparative) | 4-2 | 4-3 | 4-4 |
|---|---|---|---|---|
| Trehalose (*1) | 85.8% | 79.1% | 78.9% | 80.6% |
| Polyethylene glycol 20000 (*2) | 0.0% | 7.9% | 7.9% | 8.1% |
| Sodium hyaluronate (*3) | 0.9% | 0.8% | 0.8% | 0.8% |
| Tamarind gum (*4) | 0.4% | 0.4% | 0.6% | 0.4% |
| Polyquaternium-51 (*5) | 4.3% | 4.0% | 3.9% | 2.0% |
| PEG/PPG-1417 dimethyl ether | 8.6% | 7.9% | 7.9% | 8.1% |
| Formability | B | A | A | A |
| Releasability | B | B | C | C |
| Resolubility | B | B | B | B |
| Usability | B | A | A | A |

(*3) Biohyalo 12; manufactured by Shiseido Company, Ltd.
(*4) GLYLOID 6C; manufactured by Dainippon Pharmaceutical Co., Ltd.
(*5) Lipdure-PMB (Ph10); manufactured by NOF Corporation

**[Table 5]**

| Test Example | 5-1 (comparative) | 5-2 (comparative) | 5-3 | 5-4 | 5-5 |
|---|---|---|---|---|---|
| Trehalose (*1) | 97.1% | 75.2% | 65.4% | 65.1% | 67.6% |
| Polyethylene glycol 20000 (*2) | - | - | 13.1% | 13.0% | 13.5% |
| Sodium hyaluronate (*3) | 1.9% | 1.5% | 1.3% | 1.3% | 1.4% |
| Tamarind gum (*4) | 1.0% | 0.8% | 0.7% | 1.0% | 0.7% |
| Polyquaternium-51 (*5) | - | 7.5% | 6.5% | 6.5% | 3.4% |
| PEG/PPG-14/7 dimethyl | - | 15.0% | 13.1% | 13.0% | 13.5% |
| ether | | | | | |
| Formability | D | C | A | B | B |
| Releasability | B | B | B | B | B |
| Resolubility | B | B | B | B | B |
| Usability | C | B | B | A | A |

From Tables 4 and 5, in order to enhance usability of the tablet cosmetic, it was found that usability can be enhanced while formability, releasability and resolubility are maintained by blending the polyethylene glycol (B) and further adding the moisturizer and thickener.

Next, the number average molecular weight of the polyethylene glycol (B) to be blended was investigated.

**[Table 6] - Test Examples 6-1, 6-2, 6-3, 6-4 and 6-5 are comparative examples.**

| Test Examples | 6-1 | 6-2 | 6-3 | 6-4 | 6-5 | 6-6 | 6-7 |
|---|---|---|---|---|---|---|---|
| Trehalose (*1) | 67.3% | 67.3% | 67.3% | 67.3% | 67.3% | 67.3% | 67.3% |
| Polyethylene glycol 300 | 13.5% | - | - | - | - | - | - |
| Polyethylene glycol 1000 | - | 13.5% | - | - | - | - | - |
| Polyethylene glycol 1500 | - | - | 13.5% | - | - | - | - |
| Polyethylene glycol 4000 | - | - | - | 13.5% | - | - | - |
| Polyethylene glycol 6000 | - | - | - | - | 13.5% | - | - |
| Polyethylene glycol 11000 | - | - | - | - | - | 13.5% | - |
| Polyethylene glycol 20000 | - | - | - | - | - | - | 13.5% |
| Sodium hyaluronate (*3) | 1.3% | 1.3% | 1.3% | 1.3% | 1.3% | 1.3% | 1.3% |
| Tamarind gum (*4) | 1.0% | 1.0% | 1.0% | 1.0% | 1.0% | 1.0% | 1.0% |
| Polyquaternium-51 (*5) | 3.4% | 3.4% | 3.4% | 3.4% | 3.4% | 3.4% | 3.4% |
| PEG/PPG-14/7 dimethyl ether | 13.5% | 13.5% | 13.5% | 13.5% | 13.5% | 13.5% | 13.5% |
| Formability | D | D | D | A | A | A | A |
| Releasability | C | C | C | B | B | A | A |
| Resolubility | B | B | B | B | B | B | B |
| Usability | A | A | A | A | A | A | A |
| Bulk density (g/ml) | 0.13 | 0.14 | 0.14 | 0.14 | 0.14 | 0.15 | 0.15 |

From Test Examples 6-1 to 6-7, the number average molecular weight of the polyethylene glycol (B) is preferably 4000 to 20000 in terms of formability and usability.

**[Table 7-1]**

| Test Examples | 7-1 | 7-2 | 7-3 | 7-4 | 7-5 | 7-6 |
|---|---|---|---|---|---|---|
| Trehalose | 73.2% | 76.4% | 73.7% | 71.4% | 74.8% | 71.9% |
| Polyethylene glycol 20000 | 8.4% | 4.4% | 8.4% | 8.9% | 4.7% | 9.0% |
| Sodium hyaluronate | 0.8% | 0.9% | 0.2% | 0.9% | 0.9% | 0.2% |
| Xanthan gum | 0.2% | 0.2% | 0.2% | 0.2% | 0.2% | 0.2% |
| PEG/PPG-14/7 dimethyl ether | 8.4% | 8.7% | 8.4% | 8.9% | 9.3% | 9.0% |
| PPG-13 decyltetradeceth-24 | 2.3% | 2.4% | 2.3% | 2.5% | 2.6% | 2.5% |
| Water-soluble collagen | 4.2% | 4.4% | 4.2% | 4.5% | 4.7% | 4.5% |
| Polyquaternium-51 | 2.1% | 2.2% | 2.1% | 2.2% | 2.3% | 2.2% |
| Citric acid | 0.1% | 0.1% | 0.1% | 0.1% | 0.1% | 0.1% |
| Sodium citrate | 0.3% | 0.3% | 0.3% | 0.4% | 0.4% | 0.4% |
| Perfume | Suitable amount | Suitable amount | Suitable amount | Suitable amount | Suitable amount | Suitable amount |

Test Examples 7-1 to 7-6 were tablet cosmetics excellent in resolubility and usability, and also excellent in high-temperature stability.

Further described is a formulation example of a tablet-type freeze-dried cosmetic (detergent) as follows.

**[Table 8]**

| Formulation Example 1 (comparative) | Blending Amount (%) |
|---|---|
| Trehalose | 37.2% |
| PEG-400 | 4.3% |
| Sodium hyaluronate | 0.4% |
| Xanthan gum | 0.2% |
| Potassium Cocoyl Glutamate | 42.5% |
| Sodium Methyl Cocoyl Taurate | 2.1% |
| Polyglyceryl-4 Lauryl Ether | 10.6% |
| PEG-60 Hydrogenated Castor Oil | 1.1% |
| PEG-60 Glyceryl Isostearate | 1.1% |
| Triethylhexanoin | 0.1% |
| Citric acid | 0.2% |
| Sodium Citrate | 0.2% |
| Perfume | Suitable amount |

Further described is a formulation example of a tablet-type freeze-dried cosmetic (hair treatment) as follows.

**[Table 9]**

| Formulation Example 2 (comparative) | Blending Amount (%) |
|---|---|
| Trehalose | 56.3% |
| PEG-400 | 1.6% |
| Behentrimonium Chloride | 6.4% |
| Steartrimonium Chloride | 3.9% |
| Behenyl alcohol | 3.2% |
| Stearyl alcohol | 6.4% |
| Cetanol | 4.8% |
| Vaseline | 1.6% |
| Dimethicone | 6.4% |
| Aminopropyl Dimethicone | 6.4% |
| Amodimethicone | 0.6% |
| Phenoxyethanol | 1.6% |
| Citric acid | 0.3% |
| Sodium Citrate | 0.3% |
| Perfume | Suitable amount |

Further described is a formulation example of a tablet-type freeze-dried cosmetic (shampoo) as follows.

**Table 10**

| Formulation Example 3 (comparative) | Blending Amount (%) |
|---|---|
| Trehalose | 31.5% |
| PEG-400 | 1.3% |
| Sodium Laureth Sulfate | 39.4% |
| Sodium Methyl Cocoyl Taurate | 5.3% |
| Cocamidopropyl Betaine | 13.1% |
| Polyquatemium-10 | 0.8% |
| Polyquatemium-7 | 0.1% |
| Coconut Fatty Acid Monoethanolamide | 1.3% |
| Ethyleneglycol Distearate | 3.9% |
| Table salt | 2.6% |
| Citric acid | 0.3% |
| Sodium Citrate | 0.3% |
| Perfume | Suitable amount |

Further described ar formulation examples 4-7 of a tablet-type freeze-dried cosmetic (cosmetic for skin application) as follows.

**[Table 11]**

| Formulation Example 4 (comparative) | Blending Amount (%) |
|---|---|
| Trehalose | 85.2% |
| PEG-400 | 10.0% |
| Sodium Hyaluronate | 0.5% |
| Xanthan gum | 0.3% |
| PPG-13 decvltetradeceth-24 | 2.8% |
| Inositol | 0.1% |
| Magnesium Ascorbyl Phosphate | 0.1% |
| Citric acid (food) | 0.1% |
| Sodium Citrate | 0.4% |
| Methylparaben | 0.7% |
| Perfume | Suitable amount |

**[Table 12]**

| Formulation Example 5 (comparative) | Blending Amount (%) |
|---|---|
| Trehalose | 85.1% |
| PEG-400 | 10.0% |
| Sodium Hyaluronate | 0.5% |
| Xanthan gum | 0.3% |
| PPG-13 decyltetradeceth-24 | 2.8% |
| Ginkgo Biloba Leaf Extract | 0.1% |
| Curcuma Longa (Turmeric) Root Extract | 0.1% |
| Sapindus Mulcorossi Peel Extract | 0.1% |
| Citric acid (food) | 0.1% |
| Sodium Citrate | 0.4% |
| Methylparaben | 0.7% |
| Perfume | Suitable amount |

**[Table 13]**

| Formulation Example 6 (comparative) | Blending Amount (%) |
|---|---|
| Trehalose | 82.8% |
| PEG-400 | 9.7% |
| Sodium Hyaluronate | 0.5% |
| Xanthan gum | 0.2% |
| PPG-13 decyltetradeceth-24 | 2.7% |
| 2- (Methacryloyloxy) ethyl phosphorylcholine/butyl methacrylate copolymer | 2.4% |
| Water-soluble collagen | 0.5% |
| Calcium Chloride | 0.0% |
| Magnesium Chloride | 0.0% |
| Citric acid (food) | 0.1% |
| Sodium Citrate | 0.4% |
| Methylparaben | 0.7% |
| Perfume | Suitable amount |

**[Table 14]**

| Formulation Example 7 (comparative) | Blending Amount (%) |
|---|---|
| Trehalose | 80.8% |
| PEG-400 | 9.5% |
| Sodium Hyaluronate | 0.5% |
| Xanthan gum | 0.2% |
| PPG-13 decyltetradeceth-24 | 2.6% |
| Allantoin | 0.5% |
| Tranexamic acid | 4.8% |
| Citric acid (food) | 0.1% |
| Sodium Citrate | 0.4% |
| Methylparaben | 0.7% |
| Perfume | Suitable amount |

## Claims

1. A freeze-dried tablet cosmetic comprising:
(A) 30 to 91% by mass of trehalose;
(B) 4.0 to 16% by mass of polyethylene glycol having a molecular weight of 11000 to 25000;
(C) 0.01 to 2% by mass of hyaluronic acid; and
(D) 0.01 to 1.0% by mass of a thickener selected from a group consisting of xanthan gum, tamarind gum, sodium alginate, gellan gum, agar, polyvinyl alcohol, carboxyvinyl polymer, and quince seed,
wherein a bulk specific gravity of said cosmetic is 0.1 to 0.5 g/ml.

2. The freeze-dried tablet cosmetic according to claim 1, wherein: said (B) comprises said polyethylene glycol having a number average molecular weight of 15000 to 25000.

## Patentansprüche

1. Gefriergetrocknetes Kosmetikum in Tablettenform, umfassend:
(A) 30 bis 91 Masse-% Trehalose;
(B) 4,0 bis 16 Masse-% Polyethylenglykol mit einem Molekulargewicht von 11000 bis 25000;
(C) 0,01 bis 2 Masse-% Hyaluronsäure; und
(D) 0,01 bis 1,0 Masse-% eines Verdickungsmittels, ausgewählt aus einer Gruppe, bestehend aus Xanthangummi, Tamarindengummi, Natriumalginat, Gellangummi, Agar, Polyvinylalkohol, Carboxyvinylpolymer und Quittensamen,
wobei das spezifische Schüttgewicht des Kosmetikums 0,1 bis 0,5 g/ml beträgt.

2. Gefriergetrocknetes Kosmetikum in Tablettenform nach Anspruch 1, wobei: das (B) das Polyethylenglykol mit einem zahlenmittleren Molekulargewicht von 15000 bis 25000 umfasst.

## Revendications

1. Cosmétique en comprimé lyophilisé comportant :
(A) 30 à 91 % en masse de tréhalose ;
(B) 4,0 à 16 % en masse de polyéthylène glycol ayant une masse moléculaire de 11 000 à 25 000 ;
(C) 0,01 à 2 % en masse d'acide hyaluronique ; et
(D) 0,01 à 1,0 % en masse d'un épaississant choisi parmi un groupe constitué de gomme de xanthane, de gomme de tamarin, d'alginate de sodium, de gomme de gellane, d'agar, d'alcool polyvinylique, de polymère carboxyvinylique et de graine de coing,
dans lequel une densité apparente dudit cosmétique est de 0,1 à 0,5 g/ml.

2. Cosmétique en comprimé lyophilisé selon la revendication 1, dans lequel ledit composé (B) comporte ledit polyéthylène glycol ayant une masse moléculaire moyenne en nombre de 15 000 à 25 000.
